# EUROPÄISCHE PATENTSCHRIFT

(11) **EP 3 683 566 B1**
(45) Veröffentlichungstag und Bekanntmachung des Hinweises auf die Patenterteilung: **16.02.2022**
(21) Anmeldenummer: 20151705.9
(22) Anmeldetag: 14.01.2020
(51) Int. Cl.: G01N 21/27, G01N 21/59, G01N 33/18, G01J 3/08, G01N 21/85

(54) **VORRICHTUNG UND VERFAHREN ZUM ERFASSEN VON EIGENSCHAFTEN EINES ZU UNTERSUCHENDEN FLUIDS**
DEVICE AND METHOD FOR DETECTING PROPERTIES OF A FLUID TO BE EXAMINED
DISPOSITIF ET PROCÉDÉ DE DÉTECTION DES PROPRIÉTÉS D'UN FLUIDE À EXAMINER

(30) Priorität: 17.01.2019 AT 500372019
(43) Veröffentlichungstag der Anmeldung: 22.07.2020
(73) Patentinhaber: s::can GmbH, 1200 Wien (AT)
(72) Erfinder: BENZ, Alexander, 1140 Wien (AT); MORAWEK, Roman, 1020 Wien (AT); SPIGAHT, Bernd, 79539 Lörrach (DE); WEINGARTNER, Andreas, 2100 Korneuburg (AT)
(74) Vertreter: SONN Patentanwälte OG

(56) Entgegenhaltungen:
- DD-A1- 214 447
- DE-A1- 2 614 181
- DE-A1-102009 059 962
- DE-B4- 10 084 057

## Beschreibung

Die Erfindung betrifft eine Vorrichtung zum Erfassen von Eigenschaften eines zu untersuchenden Fluids, gemäß dem Oberbegriff von Anspruch 1.

Die Erfindung betrifft zudem ein Verfahren zum Erfassen von Eigenschaften eines zu untersuchenden Fluids gemäß dem Oberbegriff von Anspruch 10.

Vorrichtungen, insbesondere Spektrometer, und Verfahren zur Untersuchung von Inhaltsstoffen oder allgemein von Eigenschaften eines Fluids sind bekannt.

Die DE 100 84 057 B4 betrifft ein Spektrometer zur Bestimmung der Inhaltsstoffe eines gasförmigen oder flüssigen Fluides. Die Vorrichtung weist eine Lichtquelle auf, welche zumindest einen Messstrahl und zumindest einen Referenzstrahl aussendet. Der Messstrahl wird durch ein lichtdurchlässiges Fenster in das zu untersuchende Fluid und durch ein weiteres lichtdurchlässiges Fenster wieder in die Vorrichtung zurück geführt. Demgegenüber wird der Referenzstrahl im Inneren der Vorrichtung, am zu untersuchenden Fluid vorbei geführt. Sowohl der Messstrahl als auch der Referenzstrahl werden von einem Lichtdetektor empfangen. Um das Licht des Messstrahls und des Referenzstrahls einzeln erfassen zu können, ist ein Strahlselektor vorgesehen, der jeweils einen der Lichtstrahlen durchlässt und alle anderen unterbricht.

Mittels solchen Vorrichtungen die einen Messstrahl und einen Referenzstrahl nutzen, können die Eigenschaften, insbesondere die Lichtdurchlässigkeit eines zu untersuchenden Fluids, durch einen Vergleich des Messstrahls mit dem Referenzstrahl, die mit dem Lichtdetektor empfangen werden, bestimmt werden. Im Falle eines trüben Fluids werden sich die Messwerte des mit dem Lichtdetektor empfangenen Messstrahls und des mit dem Lichtdetektor empfangenen Referenzstrahls unterscheiden. Wenn das Fluid hingegen klar, d.h. besonders lichtdurchlässig ist, können die Messwerte gegebenenfalls identisch sein oder der Unterschied ist zu gering, um mit kostengünstigen Messeinrichtungen zuverlässig erfasst zu werden.

Wenn jedoch die Messwerte für den Messstrahl und den Referenzstrahl nicht zu unterscheiden sind, ist es für einen Anwender der Vorrichtung ohne geeignete Hilfseinrichtungen, d.h. nur an Hand der Messwerte, nicht nachvollziehbar, ob das Fluid tatsächlich klar, d.h. besonders lichtdurchlässig ist oder ob der Strahlselektor, welcher erst den einen und dann den anderen des Messstrahls und des Referenzstrahl zum Lichtdetektor durchlassen soll, defekt ist. Ein defekter Strahlselektor kann somit dazu führen, dass immer nur der Messstrahl oder der Referenzstrahl gemessen wird, obwohl der Strahlselektor angesteuert wird, den Messstrahl und den Referenzstrahl abwechselnd durchzulassen.

Die CN 103969206 A betrifft einen Sensor zur in-situ Erfassung einer Wasserqualität, mit einem Mikroprozessor, einer Lichtquelle, einer kollimierten konvexen Linse, zwei optischen Glasteilen, einem mechanischen optischen Schalter, einer gekoppelten konvexen Linse, einer optischen Erfassungseinheit und einem Temperatursensor, welche in einem geschlossenen Gehäuse angeordnet sind.

Die CN 109187380 A offenbart einen Wasserqualitätsdetektor mit einem ersten optischen Wegteil, einer Probenzelle, einer Referenzzelle und einem zweiten optischen Wegteil, wobei der erste optische Wegteil zum Erzeugen von parallelem Licht zur Wasserprobenerfassung verwendet wird. Die Probenzelle ist hinter dem ersten optischen Wegteil angeordnet und umfasst ein Gefäß zum Platzieren der verschmutzten Wasserprobe. Die Referenzzelle enthält einer Referenzwasserprobe und ist ebenfalls hinter dem ersten optischen Wegteil angeordnet. Der zweite optische Wegteil ist hinter der Probenzelle und der Referenzzelle angeordnet und wird zum sequentiellen Empfangen und Behandeln des durch die verschmutzte Wasserprobe und die Referenzwasserprobe dringenden Lichts verwendet.

Die JP H05-332933 A betrifft ein tragbares Messgerät zur Erfassung einer Konzentration von CO2 in Atemluft.

Die DE 10 2009 059 962 A1 offenbart einen NDIR-Zweistrahl-Gasanalysator. Dabei soll die Erkennung und Kompensation von Fehlereinflüssen, wie verschmutzungs-, alterungs- oder temperaturbedingte Veränderungen an einer Infrarot-Strahlungsquelle oder an einer Detektoranordnung vereinfacht werden. Der Gasanalysator weist eine Infrarot-Strahlungsquelle auf, welcher ein Strahlteiler zur Erzeugung eines Messstrahls durch eine Messküvette und eines Vergleichsstrahls durch eine Referenzküvette nachgeschaltet ist. Die Messküvette enthält ein zu analysierendes Gasgemisch und die Referenzküvette enthält ein Referenzgas. Zwischen dem Strahlteiler und den Küvetten ist ein Modulatorrad mit einem eine Öffnung aufweisenden Abschattungsteil vorgesehen, mit welchem der Messstrahl und der Vergleichsstrahl abwechselnd freigegeben werden. Die abwechselnd aus der Messküvette und der Referenzküvette austretende Strahlung wird mittels eines Strahlungssammlers in eine Detektoranordnung geleitet.

Die DD 214 447 betrifft die Überprüfung der Übertragungseigenschaften aller in einem Zweistrahl-Fotometer an einer Messwertbildung beteiligter Komponenten. Das Zweistrahl-Fotometer weist eine Lichtquelle auf, aus welcher ein Messsignal und ein Vergleichssignal hervorgehen, welche durch eine motorgesteuerte Chopperscheibe geführt werden. Dabei kann das Messsignal durch eine Spur a und das Vergleichssignal durch Spuren b und c der Chopperscheibe geführt werden. Die damit erhaltenen Signale treffen auf einen Empfänger dem Verstärker nachgeschaltet sind. Die verstärkten Signale werden durch einen Tiefpassfilter und einen Hochpassfilter geführt und weiter verarbeitet.

Die DE 26 14 181 A1 offenbart ein Verfahren und eine Vorrichtung zur Messung des Absorptionsverhaltens von Fluiden mittels Licht. Die Vorrichtung weist eine Lichtquelle auf, aus welcher getrennt durch eine Wand ein Messstrahl und ein Vergleichsstrahl hervorgehen, welche durch eine motorbetriebene Zerhackerscheibe geführt werden, welcher ein Lichtdetektor nachgeschaltet ist. Ziel der Vorrichtung ist es, alterungsbedingte Veränderungen der Lichtquelle und des Detektors auszugleichen. Hierfür werden in einer der Zerhackerscheibe nachgeschalteten Schaltung aus den detektierten Lichtsignalen abwechselnd ein Steuersignal und ein Messsignal erzeugt. Das Steuersignal dient einer Verstärkungsregelung, während das hierdurch korrigierte Messsignal gleichgerichtet an einem Ausgang anliegt. Die rotierende Zerhackerscheibe ist ausgebildet, abwechselnd den Messstrahl oder den Vergleichsstrahl freizugeben während der jeweils andere Strahl abgedeckt ist und in einer weiteren Position eine davon abweichende Abdeckung bereitzustellen.

Auch die sechs letztgenannten Veröffentlichungen bieten keine Lösung für eine Erkennung eines defekten Strahlselektors.

Die Aufgabe der Erfindung besteht in der Schaffung einer Vorrichtung und eines Verfahrens der eingangs genannten Art, welche(s) erkennen lässt, ob sowohl der Messstrahl als auch der Referenzstrahl freigegeben, d.h. zum Lichtdetektor durchgelassen wurden. Die Vorrichtung und das Verfahren sollen somit ermöglichen einen Defekt der Vorrichtung zu erkennen, der fehlerhafter Weise dazu führt, dass immer nur der Messstrahl oder der Referenzstrahl gemessen wird. Die Vorrichtung soll zudem platzsparend, kostengünstig und robust herstellbar sein und das Verfahren soll einfach und kostengünstig ausführbar sein.

Hierfür sieht die Erfindung eine Vorrichtung wie in Anspruch 1 und ein Verfahren wie in Anspruch 10 definiert vor. Vorteilhafte Ausführungsformen und Weiterbildungen sind in den abhängigen Ansprüchen angegeben.

Die Aufgabe wird erfindungsgemäß dadurch gelöst, dass eine dem Lichtdetektor nachgeschaltete Verarbeitungseinheit vorgesehen ist, welche zur Erkennung einer Bewegungsblockade der Abdeckeinrichtung durch einen Vergleich der Ausgangsparameter des Lichtdetektors in der ersten Position, der zweiten Position und der dritten Position der Abdeckeinrichtung ausgebildet ist. Die Vorrichtung weist eine Lichtquelle auf, die zum Aussenden mehrerer Lichtstrahlen vorgesehen ist. Die Lichtquelle weist hierfür zumindest einen Leuchtkörper, bspw. zumindest eine Xenon-Lampe, ein LED-Bauelement oder ein Laser-Bauelement auf. Somit kann bspw. die Lichtquelle einen einzigen Leuchtkörper aufweisen, dem ein Strahlteiler zur Erzeugung mehrerer Lichtstrahlen nachgeschaltet ist. Alternativ oder ergänzend kann die Lichtquelle mehrere Leuchtkörper aufweisen, die jeweils einen Lichtstrahl erzeugen. Einer der Lichtstrahlen ist ein Messstrahl, welcher für einen Durchtritt durch das Fluid vorgesehen ist und somit abhängig von den Inhaltsstoffen (oder der Trübe) des Fluids gedämpft wird. Unter einem Fluid wird ein Gas, eine Flüssigkeit oder eine Kombination von beidem verstanden. Die Intensität des Messstrahls beim Austritt aus dem Fluid hängt auch von der Intensität des Messstrahls beim Eintritt in das Fluid ab. Deshalb ist ein anderer Lichtstrahl als Referenzstrahl vorgesehen, welcher zur Umgehung des Fluids vorgesehen ist, d.h. nicht durch das Fluid geleitet wird. Der somit ungedämpfte Referenzstrahl dient in bekannter Weise einem Vergleich mit dem Messstrahl, um bspw. alterungsbedingte Reduktionen der von der Lichtquelle abgestrahlten Lichtenergie bei der Erfassung des Messstrahls berücksichtigen bzw. kompensieren zu können. Bevorzugt werden der Messstrahl und der Referenzstrahl vom selben Leuchtkörper ausgesendet. Der Lichtquelle ist eine bewegbare Abdeckeinrichtung nachgeschaltet, welche zum unterschiedlichen Abdecken der Lichtstrahlen vorgesehen ist. Hierfür ist die Abdeckeinrichtung zwischen einer ersten Position und einer zweiten Position überführbar angeordnet. Die Abdeckeinrichtung ist ausgebildet, den Messstrahl in der ersten Position freizugeben und den Referenzstrahl abzudecken und in der zweiten Position den Messstrahl abzudecken und den Referenzstrahl freizugeben. Unter einer freigebenden Position ist eine Position der Abdeckeinrichtung zu verstehen, in welcher der Lichtstrahl von der Abdeckeinrichtung zumindest teilweise durchgelassen wird, bspw. im Wesentlichen vollständig durchgelassen wird, d.h. unbeeinträchtigt ist. Die Abdeckeinrichtung ist lichtundurchlässig oder zumindest lichtdämpfend ausgebildet. Somit ist unter einer abdeckenden Position eine Position der Abdeckeinrichtung zu verstehen, in welcher der Lichtstrahl von der Abdeckeinrichtung nicht oder nur abgeschwächt durchgelassen wird. In jedem Fall wird in der freigebenden Position ein größerer Anteil vom Lichtstrahl durchgelassen als in der abdeckenden Position. In der ersten Position der Abdeckeinrichtung kann somit der Messstrahl die Abdeckeinrichtung im Wesentlichen ungehindert passieren und nach Verlassen des Fluids erfasst werden. Um Störungen des Messergebnisses zu vermeiden, ist der Referenzstrahl in dieser ersten Position abgedeckt. In der zweiten Position der Abdeckeinrichtung kann hingegen der Referenzstrahl die Abdeckeinrichtung im Wesentlichen ungehindert passieren und erfasst werden. Um Störungen des Messergebnisses des Referenzstrahls zu vermeiden, ist der Messstrahl in dieser zweiten Position abgedeckt. Um den Messstrahl und den Referenzstrahl erfassen zu können, ist, von der Lichtquelle ausgehend betrachtet, der Abdeckeinrichtung ein Lichtdetektor nachgeschaltet. Der Lichtdetektor ist ausgebildet, abhängig von der Lichtstärke oder Lichtintensität des auftreffenden Lichts einen Ausgangsparameter zu erzeugen oder zu ändern. Beispielsweise ist der Ausgangsparameter ein elektrisches Signal, welches der Lichtdetektor an seinem Ausgang bereitstellt. Der Ausgangsparameter kann aber auch ein lichtabhängiger Innenwiderstand des Lichtdetektors sein, der zwischen Anschlüssen des Lichtdetektors gemessen werden kann.

Um auf eine Bewegung bzw. Umschaltung der Abdeckeinrichtung zwischen der ersten und der zweiten Position zuverlässig rückschließen zu können, ist die Abdeckeinrichtung in eine dritte Position überführbar angeordnet. Die Abdeckeinrichtung ist ausgebildet, in der dritten Position die von der Lichtquelle ausgesendeten Lichtstrahlen im Vergleich zur ersten Position und zur zweiten Position der Abdeckeinrichtung unterschiedlich abzudecken. Die Abdeckung in der dritten Position erfolgt derart, dass der durch die Lichtstrahlen beeinflusste Ausgangsparameter des Lichtdetektors in der dritten Position unterschiedlich zum Ausgangsparameter des Lichtdetektors in der ersten Position und in der zweiten Position der Abdeckeinrichtung ist. Beispielsweise erzeugt der Lichtdetektor in der ersten, zweiten und dritten Position an einem Ausgang des Lichtdetektors unterschiedliche elektrische Spannungssignale oder sein Innenwiderstand nimmt unterschiedliche Werte an. Wesentlich ist, dass die Abdeckeinrichtung eingerichtet ist, in der dritten Position im Vergleich zur ersten und zweiten Position unterschiedliche Lichtverhältnisse am Lichtdetektor erzeugen zu können, sodass hieraus auf eine Bewegung der Abdeckeinrichtung geschlossen werden kann. Sollten die Lichtverhältnisse in den drei Positionen identisch sein, kann daher auf eine Bewegungsblockade der Abdeckeinrichtung geschlossen werden und es können Reparaturmaßnahmen ergriffen werden.

Um einen Benutzer bei der Feststellung einer Bewegungsblockade der Abdeckeinrichtung zu unterstützen, ist eine dem Lichtdetektor nachgeschaltete Verarbeitungseinheit vorgesehen, welche zur Erkennung einer Bewegungsblockade der Abdeckeinrichtung durch einen Vergleich der Ausgangsparameter des Lichtdetektors in der ersten Position, der zweiten Position und der dritten Position der Abdeckeinrichtung ausgebildet ist. Durch das Vorsehen der Verarbeitungseinheit erspart sich der Benutzer, die Ausgangsparameter, insbesondere die Werte der Ausgangsparameter, des Lichtdetektors in der ersten, zweiten und dritten Position der Abdeckeinrichtung selbst zu vergleichen. Beispielsweise vergleicht die Verarbeitungseinheit automatisch vom Lichtdetektor erzeugte elektrische Signale, die von der am Lichtdetektor auftreffenden Lichtstärke abhängen. Die Verarbeitungseinheit ist dem Lichtdetektor nachgeschaltet, d.h. sie ist mit einem Ausgang des Lichtdetektors verbunden. Die Verarbeitungseinheit kann einen Mikroprozessor oder dergleichen aufweisen. Wenn der Lichtdetektor keinen Eingang und Ausgang sondern nur zwei Anschlüsse aufweist, ist die Verarbeitungseinheit mit diesen Anschlüssen verbunden und wird ebenfalls als nachgeschaltet betrachtet. Die Verarbeitungseinheit ist ausgebildet, dann eine Bewegungsblockade zu erkennen, wenn die Ausgangsparameter (bzw. deren Werte) des Lichtdetektors in der ersten, zweiten und dritten Position der Abdeckeinrichtung identisch sind.

Es existieren zahlreiche Möglichkeiten wie die Abdeckeinrichtung in der dritten Position die Lichtstrahlen abdecken kann, um im Vergleich zur ersten und zweiten Position unterschiedliche Lichtverhältnisse am Lichtdetektor zu erzeugen. Zudem können die verschiedenen Abdeck-Möglichkeiten von den Lichtstärken der von der Lichtquelle ausgesendeten Lichtstrahlen abhängen.

Wenn im Rahmen der vorliegenden Beschreibung auf eine erste Position, eine zweite Position und/oder eine dritte Position Bezug genommen wird, so ist hierunter die erste, zweite und/oder dritte Position der Abdeckeinrichtung zu verstehen, sofern nichts Gegenteiliges angegeben wird.

Wenn weiters im Rahmen der vorliegenden Beschreibung auf eine Messung oder Erfassung des Messstrahls oder Referenzstrahls oder eines sonstigen Lichtstrahls Bezug genommen wird, so ist hierunter eine Messung oder Erfassung einer physikalischen Größe des Messstrahls, Referenzstrahls oder sonstigen Lichtstrahls, bspw. der Lichtstärke, zu verstehen. Die Messung oder Erfassung erfolgt durch den Lichtdetektor oder mit Hilfe des Lichtdetektors.

Zudem bezieht sich im Rahmen der vorliegenden Beschreibung ein Vergleich, eine Messung oder eine Differenz des/der Ausgangsparameters/Ausgangsparameter des Lichtdetektors auf den Wert des/ der Ausgangsparameters/Ausgangsparameter oder allgemein auf messbare Eigenschaften des/der Ausgangsparameters/Ausgangsparameter. Beispielsweise werden am Ausgang eines Lichtdetektors anliegende Spannungswerte oder Signalformen oder Widerstandswerte des Lichtdetektors gemessen und verglichen.

Gemäß einer bevorzugten Ausführungsform der Erfindung ist vorgesehen, dass in der dritten Position der Abdeckeinrichtung der Messstrahl oder der Referenzstrahl teilweise abgedeckt ist oder ein zusätzlicher, von der Lichtquelle ausgesendeter Lichtstrahl als zweiter Referenzstrahl, welcher zur Umgehung des Fluids vorgesehen ist, in der dritten Position der Abdeckeinrichtung im Vergleich zur ersten Position und zur zweiten Position der Abdeckeinrichtung unterschiedlich abgedeckt ist. Auf diese Weise können in der dritten Position im Vergleich zur ersten und zweiten Position unterschiedliche Lichtverhältnisse am Lichtdetektor erzeugt werden. Wenn die Lichtquelle nur den Messstrahl und den Referenzstrahl aussendet, führt die teilweise Abdeckung eines dieser Lichtstrahlen zu veränderten Lichtverhältnissen am Lichtdetektor. Beispielsweise kann sich in der dritten Position die Abdeckeinrichtung in einen Teil der Querschnittsfläche des Messstrahls oder des Referenzstrahls hinein erstrecken. Wenn hingegen ein zusätzlicher Lichtstrahl (zweiter Referenzstrahl) von der Lichtquelle ausgesendet wird, wird dieser von der Abdeckeinrichtung in der dritten Position auf eine Weise freigegeben oder zumindest teilweise abgedeckt, die sich von der Freigabe oder Abdeckung in der ersten und zweiten Position unterscheidet. Günstiger Weise ist der zweite Referenzstrahl in der ersten und zweiten Position der Abdeckeinrichtung identisch freigegeben oder identisch zumindest teilweise abgedeckt, um den Vergleich zwischen Messstrahl und Referenzstrahl nicht zu beeinflussen.

Besonders günstig ist es, wenn der zweite Referenzstrahl in der ersten Position und in der zweiten Position der Abdeckeinrichtung abgedeckt und in der dritten Position der Abdeckeinrichtung zumindest teilweise freigegeben ist. Die Abdeckung des zweiten Referenzstrahls in der ersten Position und zweiten Position der Abdeckeinrichtung verhindert eine Überlagerung des Messstrahls und des Referenzstrahls mit dem zweiten Referenzstrahl und kann somit die Genauigkeit der Messung des Messstrahls und Referenzstrahls erhöhen. In der dritten Position ist dann der zweite Referenzstrahl über zumindest einen Teil seines Querschnitts freigegeben. Die unterschiedliche Abdeckung des zweiten Referenzstrahls in der dritten Position der Abdeckeinrichtung, im Vergleich zur ersten und zweiten Position, umfasst somit auch eine Freigabe des zweiten Referenzstrahls in der dritten Position.

Gemäß einer konstruktiv besonders einfachen Ausführungsform ist die Abdeckeinrichtung verschwenkbar angeordnet. Insbesondere kann die Abdeckeinrichtung um eine Drehachse verschwenkbar angeordnet sein. Die verschwenkbar angeordnete Abdeckeinrichtung kann im Vergleich zu einer verschiebbar angeordneten Abdeckeinrichtung besonders platzsparend zwischen der ersten, zweiten und dritten Position bewegt werden. Zudem ist beim Verschwenken der Abdeckeinrichtung nur ein geringer Reibungswiderstand zu überwinden, wodurch eine unerwünschte Bewegungsblockade der Abdeckeinrichtung unwahrscheinlicher wird.

Die Bewegung der Abdeckeinrichtung kann besonders präzise erfolgen, wenn die Abdeckeinrichtung mit einem Schrittmotor verbunden ist. In diesem Fall bewegt der Schrittmotor die Abdeckeinrichtung. Der Schrittmotor kann somit Teil der Vorrichtung zum Erfassen von Eigenschaften eines zu untersuchenden Fluids sein und über eine elektrische Steuervorrichtung angesteuert werden.

Für eine besonders präzise Abdeckung und Freigabe der von der Lichtquelle ausgesendeten Lichtstrahlen kann vorgesehen sein, dass die Abdeckeinrichtung Teil eines im Pfad der Lichtstrahlen angeordneten Strahlselektors ist, welcher eine feststehende Abdeckplatte mit Durchgangsöffnungen für die Lichtstrahlen aufweist, und die Abdeckeinrichtung zur einstellbaren Abdeckung der Durchgangsöffnungen bewegbar angeordnet ist. Der Strahlselektor sorgt dafür, dass nur ausgewählte Lichtstrahlen am Lichtdetektor auftreffen können. Bevorzugt blockiert daher die Abdeckplatte des Strahlselektors, von den Durchgangsöffnungen für die Lichtstrahlen abgesehen, die Lichtausbreitung von der Lichtquelle zum Lichtdetektor. Die Durchgangsöffnungen der Abdeckplatte sind in der Ausbreitungsrichtung der Lichtstrahlen angeordnet und können mit der beweglichen Abdeckeinrichtung wahlweise freigegeben oder abgedeckt werden. Die Abdeckeinrichtung kann auch für eine teilweise Abdeckung der Durchgangsöffnungen eingerichtet sein.

Für eine besonders einfache Konstruktion kann vorgesehen sein, dass die Abdeckeinrichtung durch ein einzelnes Abdeckelement gebildet ist. Dadurch ist es nicht erforderlich, für jeden Lichtstrahl ein eigenes Abdeckelement vorzusehen.

Besonders vorteilhaft ist es, wenn das Abdeckelement durch eine in einer Ebene parallel zur Ebene der feststehenden Abdeckplatte des Strahlselektors bewegliche Platte gebildet ist, welche bewegliche Platte eine Durchgangsöffnung für einen der Lichtstrahlen aufweist. Auf diese Weise kann ein Lichtstrahl für die Passage zum Lichtdetektor freigegeben werden, indem die Durchgangsöffnung der beweglichen Platte in einer Linie mit einer der Durchgangsöffnungen der feststehenden Abdeckplatte des Strahlselektors ausgerichtet ist. Die Durchgangsöffnung der beweglichen Platte ist dabei bevorzugt ausgebildet, einen einzigen Lichtstrahl durchzulassen, während die anderen Lichtstrahlen durch die bewegliche Platte abgedeckt sind. Die bevorzugte Anordnung der beweglichen Platte in einer Ebene parallel zur Ebene der feststehenden Abdeckplatte des Strahlselektors ermöglicht eine platzsparende Konstruktion der gesamten Vorrichtung.

Wenn die Verarbeitungseinheit zur Ausgabe einer Fehlermeldung ausgebildet ist, kann der Benutzer durch die Fehlermeldung über eine erkannte Bewegungsblockade informiert werden. Die Fehlermeldung kann bspw. eines einer akustischen, optischen oder fühlbaren Fehlermeldung, ein elektrisches Signal, ein Eintrag in einer Datenbank oder eine Kombination davon sein.

Um Wiederholungen zu vermeiden bzw. zu reduzieren wird im Zusammenhang mit der folgenden Beschreibung des Verfahrens auch auf die vorangegangene Beschreibung der Vorrichtung verwiesen, soweit diese dem Sinn nach anwendbar ist. Ebenso wird in Zusammenhang mit der Beschreibung der Vorrichtung auf die folgende Beschreibung des Verfahrens verwiesen.

Verfahrensmäßig wird die Aufgabe erfindungsgemäß gelöst durch Ansteuern der beweglichen Abdeckeinrichtung zum Bewegen in eine dritte Position und Detektieren der von der Abdeckeinrichtung durchgelassenen Lichtstrahlen mit dem Lichtdetektor, in welcher dritten Position die von der Lichtquelle ausgesendeten Lichtstrahlen im Vergleich zur ersten Position und zur zweiten Position der Abdeckeinrichtung unterschiedlich abgedeckt sind, wodurch ein durch die Lichtstrahlen beeinflusster Ausgangsparameter des Lichtdetektors in der dritten Position unterschiedlich zum Ausgangsparameter des Lichtdetektors in der ersten Position und in der zweiten Position der Abdeckeinrichtung ist, und Vergleichen der Ausgangsparameter des Lichtdetektors in der ersten Position, der zweiten Position und der dritten Position der Abdeckeinrichtung mit einer dem Lichtdetektor nachgeschalteten Verarbeitungseinheit, zur Erkennung einer Bewegungsblockade der Abdeckeinrichtung. Somit werden mehrere Lichtstrahlen von einer Lichtquelle ausgesendet. Einer der Lichtstrahlen ist ein Messstrahl, welcher für einen Durchtritt durch das Fluid ausgesendet wird, d.h. der Messstrahl wird durch einen für das Fluid vorgesehenen Bereich, der bspw. außerhalb der zuvor beschriebenen Vorrichtung liegt, geführt. Ein anderer Lichtstrahl ist ein Referenzstrahl, welcher zur Umgehung des Fluids ausgesendet wird, d.h. der Referenzstrahl wird innerhalb der zuvor beschriebenen Vorrichtung, an dem für das Fluid vorgesehenen Bereich vorbei geführt. Für eine Erfassung des Messstrahls wird eine der Lichtquelle nachgeschaltete bewegbare Abdeckeinrichtung angesteuert, um sich in einem fehlerfreien Betriebsfall in eine erste den Messstrahl freigebende und den Referenzstrahl abdeckende Position zu bewegen. Für die Ansteuerung der Abdeckeinrichtung kann eine damit verbundene Steuervorrichtung vorgesehen sein. Der Abdeckeinrichtung ist ein Lichtdetektor nachgeschaltet. In dieser ersten Position der Abdeckeinrichtung werden die von der Abdeckeinrichtung in Richtung zum Lichtdetektor durchgelassenen Lichtstrahlen mit dem Lichtdetektor detektiert. Für eine Erfassung des Referenzstrahls wird die bewegliche Abdeckeinrichtung angesteuert, um sich in einem fehlerfreien Betriebsfall in eine zweite den Messstrahl abdeckende und den Referenzstrahl freigebende Position zu bewegen. In dieser zweiten Position der Abdeckeinrichtung werden die von der Abdeckeinrichtung in Richtung zum Lichtdetektor durchgelassenen Lichtstrahlen mit dem Lichtdetektor detektiert. Dabei ist es unerheblich, ob die Abdeckeinrichtung zuerst zur Bewegung in die erste Position und dann in die zweite Position oder zuerst zur Bewegung in die zweite Position und dann in die erste Position angesteuert wird. D.h. es ist unerheblich, ob zuerst der Messstrahl oder der Referenzstrahl erfasst wird.

Um feststellen zu können, ob die Abdeckeinrichtung tatsächlich in der Lage war eine Bewegung von der ersten in die zweite Position, oder umgekehrt, auszuführen, oder ob die Abdeckeinrichtung in ihrer Bewegung blockiert ist, wird die bewegliche Abdeckeinrichtung angesteuert, um sich in eine dritte Position zu bewegen. Die erste, zweite und dritte Position stellen unterschiedliche Positionen der Abdeckeinrichtung dar. In dieser dritten Position der Abdeckeinrichtung werden die von der Abdeckeinrichtung in Richtung zum Lichtdetektor durchgelassenen Lichtstrahlen mit dem Lichtdetektor detektiert. Die Vorrichtung ist derart ausgebildet, dass im fehlerfreien Betriebsfall in der dritten Position Lichtverhältnisse am Lichtdetektor anliegen, die sich von den Lichtverhältnissen in der ersten und zweiten Position unterschieden. Hierfür werden in der dritten Position die von der Lichtquelle ausgesendeten Lichtstrahlen im Vergleich zur ersten Position und zur zweiten Position unterschiedlich durch die Abdeckeinrichtung abgedeckt. Die unterschiedliche Abdeckung in der dritten Position erfolgt derart, dass ein durch die Lichtstrahlen beeinflusster Ausgangsparameter des Lichtdetektors in der dritten Position unterschiedlich zum Ausgangsparameter des Lichtdetektors in der ersten Position und in der zweiten Position der Abdeckeinrichtung ist. Die Werte (oder allgemein die Eigenschaften) des Ausgangsparameters des Lichtdetektors in der ersten Position, der zweiten Position und der dritten Position der Abdeckeinrichtung werden miteinander verglichen, um zu erkennen, ob eine Bewegungsblockade der Abdeckeinrichtung vorliegt.

Eine bevorzugte Ausführungsform des Verfahrens ist gekennzeichnet durch teilweises Abdecken des Messstrahls oder des Referenzstrahls in der dritten Position der Abdeckeinrichtung oder unterschiedliches Abdecken eines zusätzlichen, von der Lichtquelle als zweiter Referenzstrahl ausgesendeten Lichtstrahls, welcher zur Umgehung des Fluids vorgesehen ist, in der dritten Position der Abdeckeinrichtung im Vergleich zur ersten Position und zur zweiten Position der Abdeckeinrichtung. Wenn somit kein zweiter Referenzstrahl von der Lichtquelle ausgesendet wird, wird die Abdeckeinrichtung angesteuert, den Messstrahl oder den Referenzstrahl in der dritten Position teilweise abzudecken und somit unterschiedlich zur ersten und zweiten Position abzudecken. Beispielsweise wird ein Teil der Querschnittsfläche des Messstrahls oder Referenzstrahls in der dritten Position abgedeckt. Wenn hingegen ein zweiter Referenzstrahl von der Lichtquelle ausgesendet wird, wird die Abdeckeinrichtung angesteuert, diesen zweiten Referenzstrahl in der dritten Position der Abdeckeinrichtung im Vergleich zur ersten Position und zur zweiten Position der Abdeckeinrichtung unterschiedlich abzudecken. Dabei kann der zweite Referenzstrahl auch nur teilweise abgedeckt werden. Die Abdeckung in der dritten Position erfolgt jedenfalls derart, dass der Lichtdetektor im fehlerfreien Betriebsfall im Vergleich zur ersten und zweiten Position unterschiedliche Lichtverhältnisse detektiert. Auch mittels des zweiten Referenzstrahls können somit unterschiedliche Lichtverhältnisse am Lichtdetektor erzeugt werden.

Unterschiedliche Lichtverhältnisse am Lichtdetektor können beispielsweise erzielt werden durch Abdecken des zweiten Referenzstrahls in der ersten Position und in der zweiten Position der Abdeckeinrichtung und zumindest teilweises Freigeben des zweiten Referenzstrahls in der dritten Position der Abdeckeinrichtung. Somit kann die Abdeckeinrichtung angesteuert werden, den zweiten Referenzstrahl in der ersten und zweiten Position abzudecken, um die Messung des Messstrahls und des Referenzstrahls nicht zu beeinflussen, und in der dritten Position zumindest teilweise freizugeben. In der dritten Position können der Messstrahl und der Referenzstrahl so wie in der ersten oder zweiten Position oder auch anders abgedeckt sein. Wesentlich ist, dass die am Lichtdetektor auftreffenden Lichtverhältnisse in der dritten Position unterschiedlich zu den Lichtverhältnissen in der ersten

und zweiten Position sind.

Durch Vergleichen des Ausgangsparameters des Lichtdetektors in der dritten Position der Abdeckeinrichtung mit dem Ausgangsparameter des Lichtdetektors in der ersten Position oder zweiten Position der Abdeckeinrichtung in einer dem Lichtdetektor nachgeschalteten Verarbeitungseinheit und mittels der Verarbeitungseinheit Ausgeben eines Hinweises auf eine Bewegungsblockade der Abdeckeinrichtung, wenn die Differenz zwischen den verglichenen Ausgangsparametern einen vorgegebenen Schwellwert unterschreitet, kann bevorzugt eine Bewegungsblockade der Abdeckeinrichtung automatisiert erkannt werden. Dies erspart einem Benutzer eine manuelle Messung und einen manuellen Vergleich der Ausgangsparameter des Lichtdetektors. Die Verarbeitungseinheit kann somit dann einen Hinweis auf eine Bewegungsblockade der Abdeckeinrichtung ausgeben, wenn die Differenz zwischen den Werten der Ausgangsparameter des Lichtdetektors in der ersten oder zweiten Position der Abdeckeinrichtung und dem Wert des Ausgangsparameters in der dritten Position der Abdeckeinrichtung zu gering ist, d.h. einen vorgegebenen Schwellwert unterschreitet. Die Verarbeitungseinheit kann zur Eingabe bzw. Einstellung des Schwellwerts ausgebildet sein.

Günstiger Weise wird der Hinweis auf eine Bewegungsblockade der Abdeckeinrichtung erst ausgeben, wenn die Differenz zwischen den verglichenen Ausgangsparametern den vorgegebenen Schwellwert mehrmals aufeinanderfolgend unterschreitet. Auf diese Weise kann eine fehlerhafte Ausgabe eines Hinweises auf eine Bewegungsblockade, auf Grund einer kurzfristigen Störungen an der Vorrichtung, vermieden werden. Beispielsweise wird der Hinweis auf eine Bewegungsblockade erst ausgeben, wenn die genannte Differenz den Schwellwert drei mal, bevorzugt zehn mal aufeinanderfolgend unterschreitet. Der Benutzer kann somit mittels der Verarbeitungseinheit von der Bewegungsblockade informiert werden und zudem aufgefordert werden, Reparaturmaßnahmen einzuleiten.

Wenn die Lichtstrahlen in Form mehrerer Lichtblitze ausgesendet werden, kann im Vergleich zu einer kontinuierlichen Abgabe von Licht Energie gespart werden. Zudem wird die Vorrichtung hierdurch weniger erwärmt, wodurch genauere Messergebnisse des Messstrahls und des Referenzstrahls erzielt werden können. Bevorzugt werden die einzelnen Lichtblitze, die einen Lichtstrahl bilden, aufsummiert, um einen einzigen Messwert für den Lichtstrahl zu erhalten. Alternativ können die einzelnen Lichtblitze, die einen Lichtstrahl bilden, einzeln gemessen werden und deren Messwerte mit den Messwerten der Lichtblitze eines anderen Lichtstrahls verglichen werden. Der Ausgangsparameter des Lichtdetektors in der ersten, zweiten und dritten Position der Abdeckeinrichtung kann somit in jeder der drei Positionen mehrere Werte, d.h. eine Wertemenge, umfassen.

Die Vorrichtung und das Verfahren sind sowohl für die Untersuchung von Eigenschaften von Gasen als auch von Eigenschaften von Flüssigkeiten anwendbar. Besonders vorteilhaft ist jedoch eine Anwendung zur Analyse der Qualität von Gewässern.

Die Erfindung wird im Folgenden anhand von bevorzugten, nicht einschränkenden Ausführungsbeispielen unter Bezugnahme auf die Zeichnung noch weiter erläutert. Es zeigen:
Fig. 1 eine schematische Darstellung einer Vorrichtung gemäß der Erfindung;
Fig. 2 eine schematische Darstellung eines Messstrahls, eines Referenzstrahls und eines zweiten Referenzstrahls und der Abdeckeinrichtung in der ersten, zweiten und dritten Position;
Fig. 3 eine Detailansicht einer beispielhaften Abdeckeinrichtung in der ersten Position;
Fig. 4 eine Detailansicht der Abdeckeinrichtung aus Fig. 3 in der zweiten Position; und
Fig. 5 eine Detailansicht der Abdeckeinrichtung aus Fig. 3 in der dritten Position.

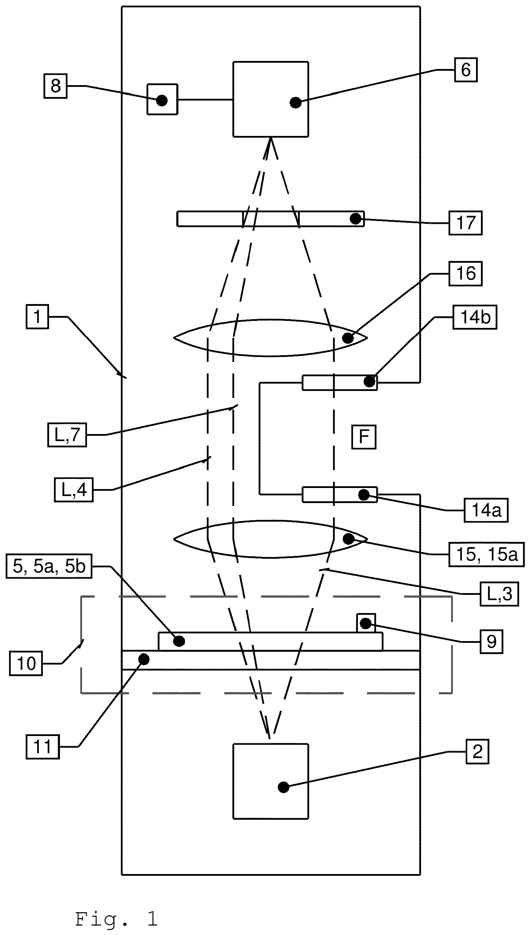

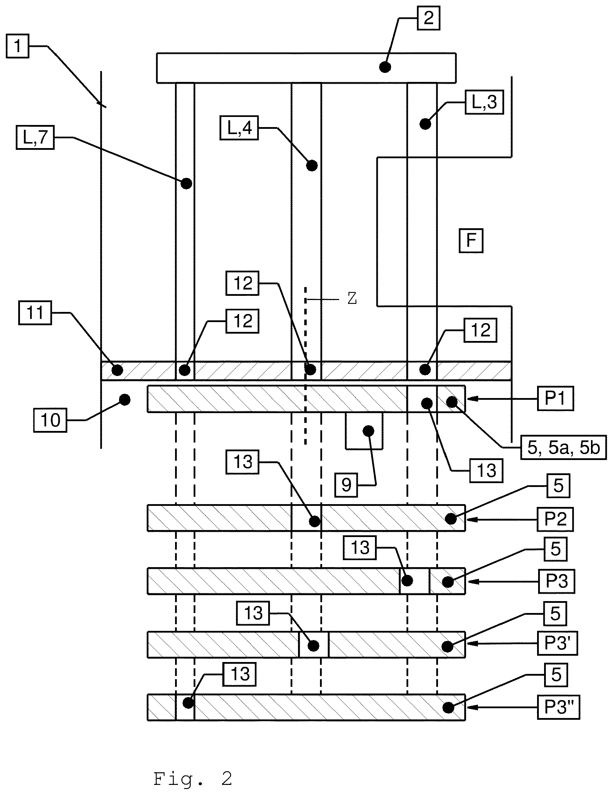

Fig. 1 zeigt eine schematische Darstellung einer beispielhaften Vorrichtung 1 zum Erfassen von Eigenschaften eines zu untersuchenden Fluids F. Das Fluid F befindet sich bevorzugt in einem Bereich außerhalb der Vorrichtung 1. Die Vorrichtung 1 weist eine Lichtquelle 2 zum Aussenden mehrerer getrennter Lichtstrahlen L auf. Einer der Lichtstrahlen L ist ein Messstrahl 3, welcher für einen Durchtritt durch das Fluid F vorgesehen ist. Hierfür kann die Vorrichtung 1 ein Lichtaustrittsfenster 14a aufweisen, durch welches der Messstrahl 3 aus der Vorrichtung 1 austritt, und ein Lichteintrittsfenster 14b aufweisen, durch welches der Messstrahl 3 nach Durchlaufen des Fluids F wieder in die Vorrichtung 1 eintritt. Ein anderer Lichtstrahl L ist ein Referenzstrahl 4, welcher zur Umgehung des Fluids F vorgesehen ist und bevorzugt innerhalb der Vorrichtung 1 geführt wird. Die Vorrichtung 1 weist zudem eine der Lichtquelle 2 nachgeschaltete bewegbare, d.h. verstellbare Abdeckeinrichtung 5 auf. Die Abdeckeinrichtung 5 ist somit im Pfad der Lichtstrahlen L angeordnet. Die Abdeckeinrichtung 5 ist zum verstellbaren Abdecken der Lichtstrahlen L, insbesondere zum Abdecken zumindest eines einzelnen Lichtstrahls L bei gleichzeitigem Freigeben zumindest eines anderen einzelnen Lichtstrahls L vorgesehen und ausgebildet. Das Abdecken und Freigeben der Lichtstrahlen L kann auch ein teilweises Abdecken und Freigeben der Lichtstrahlen L umfassen, wenn dies zweckmäßig ist. Die Abdeckeinrichtung 5 ist zwischen einer ersten den Messstrahl 3 freigebenden und Referenzstrahl 4 abdeckenden Position P1 und einer zweiten den Messstrahl 3 abdeckenden und den Referenzstrahl 4 freigebenden Position P2 überführbar angeordnet (siehe Fig. 2). Der Abdeckeinrichtung 5 ist im Beispiel gemäß Fig. 1 eine optische Einrichtung 15, bspw. eine optische Linse 15a, zur Bündelung der Lichtstrahlen L nachgeschaltet. Die Vorrichtung 1 weist zudem einen der Abdeckeinrichtung 5 nachgeschalteten Lichtdetektor 6 auf, welcher zur Erfassung der von der Abdeckeinrichtung 5 zumindest teilweise durchgelassenen, d.h. freigegebenen Lichtstrahlen L vorgesehen ist. Beispielsweise kann der Lichtdetektor 6 ein Fotowiderstand, ein Fototransistor, eine Fotodiode, ein CMOS-Sensor oder ein CCD-Sensor sein. Zwischen der Abdeckeinrichtung 5, insbesondere zwischen der optischen Einrichtung 15, und dem Lichtdetektor 6 sind bevorzugt eine optische Einrichtung 16 und eine Blende 17 zum Bündeln bzw. Lenken der Lichtstrahlen L auf den Lichtdetektor 6 angeordnet. Die Abdeckeinrichtung 5 ist zudem derart in eine dritte Position P3, P3', P3" überführbar angeordnet, in welcher die von der Lichtquelle 2 ausgesendeten Lichtstrahlen L im Vergleich zur ersten Position P1 und zur zweiten Position P2 der Abdeckeinrichtung 5 unterschiedlich abgedeckt sind. Hierdurch weist ein durch die Lichtstrahlen L beeinflusster bzw. beeinflussbarer Ausgangsparameter des Lichtdetektors 6 in der dritten Position P3, P3', P3" einen Wert/ eine Eigenschaft auf, der/die unterschiedlich zum Wert/ Eigenschaft des Ausgangsparameters des Lichtdetektors 6 in der ersten Position P1 und in der zweiten Position P2 der Abdeckeinrichtung 5 ist.

In Fig. 1 ist zudem ein zusätzlicher, von der Lichtquelle 2 ausgesendeter Lichtstrahl L als zweiter Referenzstrahl 7 strichliert dargestellt. Der zweite Referenzstrahl 7 ist optional zur Umgehung des Fluids F vorgesehen, wofür er innerhalb der Vorrichtung 1 verläuft.

Zur Bewegung der Abdeckeinrichtung 5 ist diese bevorzugt mit einem Antrieb, insbesondere einem Schrittmotor 9, verbunden und wird von diesem angetrieben.

In Fig. 1 ist weiters eine dem Lichtdetektor 6 nachgeschaltete Verarbeitungseinheit 8 dargestellt. Die Verarbeitungseinheit 8 ist zur automatischen Erkennung einer Bewegungsblockade der Abdeckeinrichtung 5 und gegebenenfalls, im Fall eine Bewegungsblockade, zur Ausgabe einer Fehlermeldung ausgebildet. Hierfür vergleicht die Verarbeitungseinheit 8 die Werte/Eigenschaften des Ausgangsparameters des Lichtdetektors 6 in der ersten Position P1, der zweiten Position P2 und der dritten Position P3, P3', P3" der Abdeckeinrichtung 5.

Die Fig. 1 und Fig. 2 zeigen schematisch eine Lichtquelle 2, aus welcher ein Messstrahl 3, ein Referenzstrahl 4 und allenfalls ein zweiter Referenzstrahl 7 als Lichtstrahlen L austreten. Die Lichtstrahlen L treffen in den dargestellten Beispielen auf einen Strahlselektor 10, der bevorzugt am Innenumfang der Vorrichtung 1 anliegt, um allfälliges am Strahlselektor 10 vorbeilaufendes Streulicht zu vermeiden. In den in Fig. 1 und Fig. 2 dargestellten Beispielen ist erkennbar, dass die Abdeckeinrichtung 5 Teil des im Pfad der Lichtstrahlen L angeordneten Strahlselektors 10 ist. Der Strahlselektor 10 weist bevorzugt eine feststehende Abdeckplatte 11 mit Durchgangsöffnungen 12 (Fig. 2) für die Lichtstrahlen L auf. Um unterschiedliche Lichtverhältnisse zu den unterschiedlichen Positionen P1, P2 und P3, P3', P3" am Lichtdetektor 6 erzeugen zu können, ist die Abdeckeinrichtung 5 zur einstellbaren Abdeckung, insbesondere zur zumindest teilweisen Abdeckung der Durchgangsöffnungen 12 bewegbar angeordnet.

Wie weiters in den Fig. 1 und 2 ersichtlich ist, kann die Abdeckeinrichtung 5 durch ein einzelnes Abdeckelement 5a gebildet sein. Das Abdeckelement 5a kann durch eine in einer Ebene parallel zur Ebene der feststehenden Abdeckplatte 11 des Strahlselektors 10 bewegliche Platte 5b gebildet sein. Die bewegliche Platte 5b weist eine Durchgangsöffnung 13 für den Durchtritt jeweils eines der Lichtstrahlen L auf.

Fig. 2 zeigt zudem die Abdeckeinrichtung 5 / das Abdeckelement 5a / die bewegliche Platte 5b in der zuvor genannten ersten, zweiten und dritten Position, P1, P2, P3, P3', P3". Insbesondere ist in der ersten Position P1 der Abdeckeinrichtung 5 der Messstrahl 3 freigegeben und der Referenzstrahl 4 abgedeckt. In der zweiten Position P2 der Abdeckeinrichtung 5 ist der Messstrahl 3 abgedeckt und der Referenzstrahl 4 freigegeben. In der dritten Position P3 der Abdeckeinrichtung 5 ist der Messstrahl 3 teilweise abgedeckt und der Referenzstrahl 4 abgedeckt. In einer anderen dritten Position P3' der Abdeckeinrichtung 5 ist der Messstrahl 3 abgedeckt und der Referenzstrahl 4 teilweise abgedeckt. In den beispielhaften Positionen P1, P2, P3 und P3' ist zudem der zweite Referenzstrahl 7 abgedeckt. Demnach könnte in den beispielhaften Positionen P1, P2, P3 und P3' der zweite Referenzstrahl 7 auch weggelassen werden. In einer weiteren dritten Position P3" der Abdeckeinrichtung 5 sind der Messstrahl 3 und Referenzstrahl 4 abgedeckt und der zweite Referenzstrahl 7 ist freigegeben. In Position P3" könnte der zweite Referenzstrahl 7 auch nur teilweise freigegeben sein.

Somit können in der dritten Position P3, P3' der Abdeckeinrichtung 5 der Messstrahl 3 oder der Referenzstrahl 4 teilweise abgedeckt sein. Oder es kann in der dritten Position P3" der Abdeckeinrichtung 5 ein zusätzlicher, von der Lichtquelle 2 ausgesendeter Lichtstrahl L als zweiter Referenzstrahl 7, welcher zur Umgehung des Fluids F vorgesehen ist, im Vergleich zur ersten Position P1 und zur zweiten Position P2 der Abdeckeinrichtung 5 unterschiedlich abgedeckt sein. Dabei kann der zweite Referenzstrahl 7 in der ersten Position P1 und in der zweiten Position P2 der Abdeckeinrichtung abgedeckt und in der dritten Position P3" der Abdeckeinrichtung 5 zumindest teilweise freigegeben sein.

Aus der Beschreibung wird offensichtlich, dass zahlreiche Kombinationsmöglichkeiten zur zumindest teilweisen Abdeckung und Freigabe des Messstrahls 3, des Referenzstrahls 4 und des allfälligen zweiten Referenzstrahls 7 existieren. Wesentlich ist, dass in der dritten Position P3, P3', P3" am Lichtdetektor 6 andere Lichtverhältnisse als in der ersten Position P1 und zweiten Position P2 vorliegen.

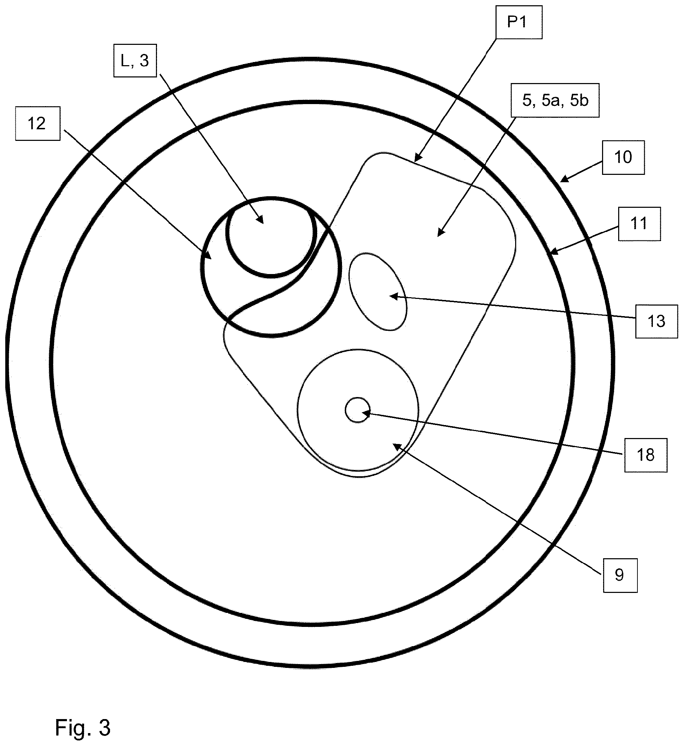

Fig. 3 zeigt eine Detailansicht einer beispielhaften Abdeckeinrichtung 5 in der ersten Position P1. Die Abdeckeinrichtung 5 ist bevorzugt über eine Schwenkachse 18 verschwenkbar in der Vorrichtung 1 angeordnet. Auch in Fig. 3 ist erkennbar, dass die Abdeckeinrichtung 5 Teil eines im Pfad der Lichtstrahlen L angeordneten Strahlselektors 10 sein kann, dass der Strahlselektor 10 eine feststehende Abdeckplatte 11 mit Durchgangsöffnungen 12 für die Lichtstrahlen L aufweisen kann, und dass die Abdeckeinrichtung 5 zur einstellbaren Abdeckung der Durchgangsöffnungen 12 bewegbar angeordnet sein kann. Im dargestellten Beispiel ist die Abdeckeinrichtung 5 durch ein einzelnes Abdeckelement 5a gebildet, welches durch eine bewegliche Platte 5b mit einer Durchgangsöffnung 13 für jeweils einen der Lichtstrahlen L gebildet ist. Insbesondere ist in der ersten Position P1 der Abdeckeinrichtung 5 der Messstrahl 3 freigegeben, da die Abdeckeinrichtung 5 vom Messstrahl 3 weggeschwenkt ist, und der Referenzstrahl 4 ist durch die Abdeckeinrichtung 5 abgedeckt.

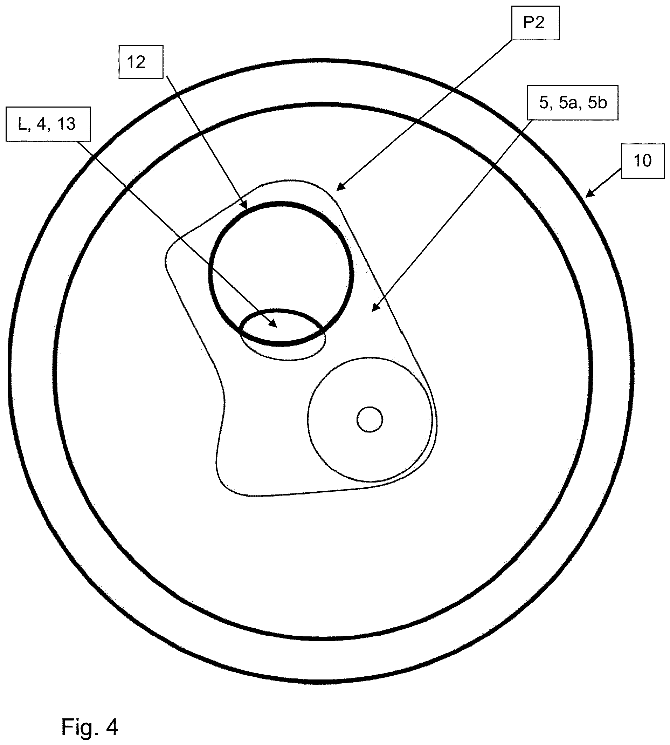

Fig. 4 zeigt eine Detailansicht in welcher die Abdeckeinrichtung 5 in die zweiten Position P2 bewegt, insbesondere verschwenkt wurde. In dieser zweiten Position P2 ist der Messstrahl 3 durch die Abdeckeinrichtung 5 abgedeckt und der Referenzstrahl 4 ist freigegeben, da die Durchgangsöffnung 13 der beweglichen Platte 5b und der Referenzstrahl 4 im Wesentlichen in einer Linie angeordnet sind.

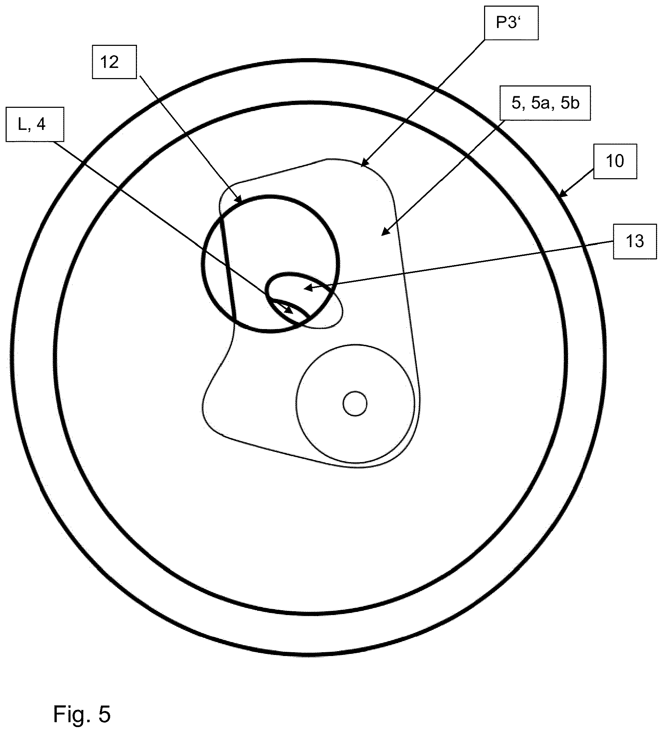

Fig. 5 zeigt eine weitere Detailansicht in welcher die Abdeckeinrichtung 5 in die dritte Position P3' bewegt, insbesondere verschwenkt wurde. In dieser dritten Position P3' ist der Messstrahl 3 durch die Abdeckeinrichtung 5 abgedeckt und der Referenzstrahl 4 ist teilweise freigegeben, da die Durchgangsöffnung 13 der beweglichen Platte 5b und der Referenzstrahl 4 einander teilweise überdecken.

## Patentansprüche

1. Vorrichtung (1) zum Erfassen von Eigenschaften eines zu untersuchenden Fluids (F), mit einer Lichtquelle (2) zum Aussenden mehrerer Lichtstrahlen (L), von welchen einer der Lichtstrahlen (L) ein Messstrahl (3) ist, welcher für einen Durchtritt durch das Fluid (F) vorgesehen ist, und ein anderer Lichtstrahl (L) ein Referenzstrahl (4) ist, welcher zur Umgehung des Fluids (F) vorgesehen ist, mit einer der Lichtquelle (2) nachgeschalteten bewegbaren Abdeckeinrichtung (5), welche zum Abdecken der Lichtstrahlen (L) vorgesehen ist und die zwischen einer ersten den Messstrahl (3) freigebenden und Referenzstrahl (4) abdeckenden Position (P1) und einer zweiten den Messstrahl (3) abdeckenden und den Referenzstrahl (4) freigebenden Position (P2) überführbar angeordnet ist, und mit einem der Abdeckeinrichtung (5) nachgeschalteten Lichtdetektor (6), wobei die Abdeckeinrichtung (5) in eine dritte Position (P3, P3', P3") überführbar angeordnet ist, in welcher die von der Lichtquelle (2) ausgesendeten Lichtstrahlen (L) im Vergleich zur ersten Position (P1) und zur zweiten Position (P2) der Abdeckeinrichtung (5) unterschiedlich abgedeckt sind, wodurch ein durch die Lichtstrahlen (L) beeinflusster Ausgangsparameter des Lichtdetektors (6) in der dritten Position (P3, P3', P3'') unterschiedlich zum Ausgangsparameter des Lichtdetektors (6) in der ersten Position (P1) und in der zweiten Position (P2) der Abdeckeinrichtung (5) ist, **dadurch gekennzeichnet, dass** eine dem Lichtdetektor (6) nachgeschaltete Verarbeitungseinheit (8) vorgesehen ist, welche zur Erkennung einer Bewegungsblockade der Abdeckeinrichtung (5) durch einen Vergleich der Ausgangsparameter des Lichtdetektors (6) in der ersten Position (P1), der zweiten Position (P2) und der dritten Position (P3, P3', P3'') der Abdeckeinrichtung (5) ausgebildet ist.

2. Vorrichtung (1) nach Anspruch 1, **dadurch gekennzeichnet, dass** in der dritten Position (P3, P3') der Abdeckeinrichtung (5) der Messstrahl (3) oder der Referenzstrahl (4) teilweise abgedeckt ist oder ein zusätzlicher, von der Lichtquelle (2) ausgesendeter Lichtstrahl (L) als zweiter Referenzstrahl (7), welcher zur Umgehung des Fluids (F) vorgesehen ist, in der dritten Position (P3") der Abdeckeinrichtung (5) im Vergleich zur ersten Position (P1) und zur zweiten Position (P2) der Abdeckeinrichtung (5) unterschiedlich abgedeckt ist.

3. Vorrichtung (1) nach Anspruch 2, **dadurch gekennzeichnet, dass** der zweite Referenzstrahl (7) in der ersten Position (P1) und in der zweiten Position (P2) der Abdeckeinrichtung (5) abgedeckt und in der dritten Position (P3") der Abdeckeinrichtung (5) zumindest teilweise freigegeben ist.

4. Vorrichtung (1) nach einem der Ansprüche 1 bis 3, **dadurch gekennzeichnet, dass** die Abdeckeinrichtung (5) verschwenkbar angeordnet ist.

5. Vorrichtung (1) nach einem der Ansprüche 1 bis 4, **dadurch gekennzeichnet, dass** die Abdeckeinrichtung (5) mit einem Schrittmotor (9) verbunden ist.

6. Vorrichtung (1) nach einem der Ansprüche 1 bis 5, **dadurch gekennzeichnet, dass** die Abdeckeinrichtung (5) Teil eines im Pfad der Lichtstrahlen (L) angeordneten Strahlselektors (10) ist, welcher eine feststehende Abdeckplatte (11) mit Durchgangsöffnungen (12) für die Lichtstrahlen (L) aufweist, und die Abdeckeinrichtung (5) zur einstellbaren Abdeckung der Durchgangsöffnungen (12) bewegbar angeordnet ist.

7. Vorrichtung (1) nach einem der Ansprüche 1 bis 6, **dadurch gekennzeichnet, dass** die Abdeckeinrichtung (5) durch ein einzelnes Abdeckelement (5a) gebildet ist.

8. Vorrichtung (1) nach Anspruch 6 und 7, **dadurch gekennzeichnet, dass** das Abdeckelement (5a) durch eine in einer Ebene parallel zur Ebene der feststehenden Abdeckplatte (11) des Strahlselektors (10) bewegliche Platte (5b) gebildet ist, welche bewegliche Platte (5b) eine Durchgangsöffnung (13) für einen der Lichtstrahlen (L) aufweist.

9. Vorrichtung (1) nach einem der Ansprüche 1 bis 8, **dadurch gekennzeichnet, dass** die Verarbeitungseinheit (8) zur Ausgabe einer Fehlermeldung ausgebildet ist.

10. Verfahren zum Erfassen von Eigenschaften eines zu untersuchenden Fluids (F), durch Aussenden mehrerer Lichtstrahlen (L) von einer Lichtquelle (2), von welchen einer der Lichtstrahlen (L) ein Messstrahl (3) ist, welcher für einen Durchtritt durch das Fluid (F) ausgesendet wird, und ein anderer Lichtstrahl (L) ein Referenzstrahl (4) ist, welcher zur Umgehung des Fluids (F) ausgesendet wird, Ansteuern einer der Lichtquelle (2) nachgeschalteten bewegbaren Abdeckeinrichtung (5) zum Bewegen in eine erste den Messstrahl (3) freigebende und den Referenzstrahl (4) abdeckende Position (P1) und Detektieren der von der Abdeckeinrichtung (5) durchgelassenen Lichtstrahlen (L) mit einem der Abdeckeinrichtung (5) nachgeschalteten Lichtdetektor (6) und Ansteuern der beweglichen Abdeckeinrichtung (5) zum Bewegen in eine zweite den Messstrahl (3) abdeckende und den Referenzstrahl (4) freigebende Position (P2) und Detektieren der von der Abdeckeinrichtung (5) durchgelassenen Lichtstrahlen (L) mit dem Lichtdetektor (6), **gekennzeichnet durch** Ansteuern der beweglichen Abdeckeinrichtung (5) zum Bewegen in eine dritte Position (P3, P3', P3") und Detektieren der von der Abdeckeinrichtung (5) durchgelassenen Lichtstrahlen (L) mit dem Lichtdetektor (6), in welcher dritten Position (P3, P3', P3") die von der Lichtquelle (2) ausgesendeten Lichtstrahlen (L) im Vergleich zur ersten Position (P1) und zur zweiten Position (P2) der Abdeckeinrichtung (5) unterschiedlich abgedeckt sind, wodurch ein durch die Lichtstrahlen (L) beeinflusster Ausgangsparameter des Lichtdetektors (6) in der dritten Position (P3, P3', P3") unterschiedlich zum Ausgangsparameter des Lichtdetektors (6) in der ersten Position (P1) und in der zweiten Position (P2) der Abdeckeinrichtung (5) ist, und Vergleichen der Ausgangsparameter des Lichtdetektors (6) in der ersten Position (P1), der zweiten Position (P2) und der dritten Position (P3, P3', P3'') der Abdeckeinrichtung (5) mit einer dem Lichtdetektor (6) nachgeschalteten Verarbeitungseinheit (8), zur Erkennung einer Bewegungsblockade der Abdeckeinrichtung (5).

11. Verfahren nach Anspruch 10, **gekennzeichnet durch** teilweises Abdecken des Messstrahls (3) oder des Referenzstrahls (4) in der dritten Position (P3, P3') der Abdeckeinrichtung (5) oder unterschiedliches Abdecken eines zusätzlichen, von der Lichtquelle (2) als zweiter Referenzstrahl (7) ausgesendeten Lichtstrahls (L), welcher zur Umgehung des Fluids (F) vorgesehen ist, in der dritten Position (P3") der Abdeckeinrichtung (5) im Vergleich zur ersten Position (P1) und zur zweiten Position (P2) der Abdeckeinrichtung (5).

12. Verfahren nach Anspruch 11, **gekennzeichnet durch** Abdecken des zweiten Referenzstrahls (7) in der ersten Position (P1) und in der zweiten Position (P2) der Abdeckeinrichtung (5) und zumindest teilweises Freigeben des zweiten Referenzstrahls (7) in der dritten Position (P3") der Abdeckeinrichtung (5).

13. Verfahren nach einem der Ansprüche 10 bis 12, **gekennzeichnet durch** Vergleichen des Ausgangsparameters des Lichtdetektors (6) in der dritten Position (P3, P3', P3'') der Abdeckeinrichtung (5) mit dem Ausgangsparameter des Lichtdetektors (6) in der ersten Position (P1) oder zweiten Position (P2) der Abdeckeinrichtung (5) in einer dem Lichtdetektor (6) nachgeschalteten Verarbeitungseinheit (8) und mittels der Verarbeitungseinheit (8) Ausgeben eines Hinweises auf eine Bewegungsblockade der Abdeckeinrichtung (5), wenn die Differenz zwischen den verglichenen Ausgangsparametern einen vorgegebenen Schwellwert unterschreitet.

14. Verfahren nach Anspruch 13, **gekennzeichnet durch** Ausgeben des Hinweises auf eine Bewegungsblockade der Abdeckeinrichtung (5) erst, wenn die Differenz zwischen den verglichenen Ausgangsparametern den vorgegebenen Schwellwert mehrmals aufeinanderfolgend unterschreitet.

## Claims

1. Apparatus (1) for detecting properties of a fluid (F) to be examined, comprising a light source (2) for emitting a plurality of light beams (L), of which one of the light beams (L) is a measuring beam (3) which is intended to pass through the fluid (F), and another light beam (L) is a reference beam (4) which is intended to bypass the fluid (F), comprising a movable cover device (5) arranged downstream of the light source (2), which device is intended to cover the light beams (L) and which is arranged so as to be transferrable between a first position (P1) which exposes the measuring beam (3) and covers the reference beam (4) and a second position (P2) which covers the measuring beam (3) and exposes the reference beam (4) and comprising a light detector (6) arranged downstream of the cover device (5), the cover device (5) being arranged so as to be transferrable into a third position (P3, P3', P3") in which the light beams (L) emitted by the light source (2) are covered differently in comparison to the first position (P1) and to the second position (P2) of the cover device (5), whereby an output parameter of the light detector (6) influenced by the light beams (L) in the third position (P3, P3', P3") differs from the output parameter of the light detector (6) in the first position (P1) and in the second position (P2) of the cover device (5), **characterised in that** a processing unit (8) arranged downstream of the light detector (6) is provided, which unit is designed to detect a blockage in the movement of the cover device (5) by comparing the output parameters of the light detector (6) in the first position (P1), the second position (P2) and the third position (P3, P3', P3") of the cover device (5).

2. Apparatus (1) according to claim 1, **characterised in that** in the third position (P3, P3') of the cover device (5), the measuring beam (3) or the reference beam (4) is partially covered or an additional light beam (L) emitted by the light source (2) as a reference beam (7), which light beam is intended to bypass the fluid (F), is covered differently in the third position (P3") of the cover device (5) in comparison to the first position (P1) and to the second position (P2) of the cover device (5).

3. Apparatus (1) according to claim 2, **characterised in that** the second reference beam (7) is covered in the first position (P1) and in the second position (P2) of the cover device (5) and is at least partially exposed in the third position (P3") of the cover device (5).

4. Apparatus (1) according to any of claims 1 to 3, **characterised in that** the cover device (5) is arranged so as to be pivotable.

5. Apparatus (1) according to any of claims 1 to 4, **characterised in that** the cover device (5) is connected to a stepping motor (9).

6. Apparatus (1) according to any of claims 1 to 5, **characterised in that** the cover device (5) is part of a beam selector (10) which is arranged in the path of the light beams (L) and which has a fixed cover plate (11) having holes (12) for the light beams (L), and the cover device (5) is arranged so as to be movable in order to adjustably cover the holes (12).

7. Apparatus (1) according to any of claims 1 to 6, **characterised in that** the cover device (5) is formed by a single cover element (5a).

8. Apparatus (1) according to either claim 6 or claim 7, **characterised in that** the cover element (5a) is formed by a plate (5b) which is movable in a plane parallel to the plane of the fixed cover plate (11) of the beam selector (10), which movable plate (5b) has a hole (13) for one of the light beams (L).

9. Apparatus (1) according to any of claims 1 to 8, **characterised in that** the processing unit (8) is designed to output an error message.

10. Method for detecting properties of a fluid (F) to be examined by emitting a plurality of light beams (L) from a light source (2), of which one of the light beams (L) is a measuring beam (3) which is emitted to pass through the fluid (F), and another light beam (L) is a reference beam (4) which is emitted to bypass the fluid (F), by controlling a movable cover device (5) arranged downstream of the light source (2) to move it into a first position (P1) which exposes the measuring beam (3) and covers the reference beam (4) and by detecting the light beams (L) let through by the cover device (5) using a light detector (6) arranged downstream of the cover device (5) and by controlling the movable cover device (5) to move it into a second position (P2) which covers the measuring beam (3) and exposes the reference beam (4) and by detecting the light beams (L) let through by the cover device (5) using the light detector (6), **characterised by** controlling the movable cover device (5) to move it into a third position (P3, P3', P3") and detecting the light beams (L) let through by the cover device (5) using the light detector (6), in which third position (P3, P3', P3") the light beams (L) emitted by the light source (2) are covered differently in comparison to the first position (P1) and to the second position (P2) of the cover device (5), whereby an output parameter of the light detector (6) influenced by the light beams (L) in the third position (P3, P3', P3") differs from the output parameter of the light detector (6) in the first position (P1) and in the second position (P2) of the cover device (5), and **characterised by** comparing the output parameters of the light detector (6) in the first position (P1), the second position (P2) and the third position (P3, P3', P3") of the cover device (5) using a processing unit (8) for detecting a blockage in the movement of the cover device (5), which unit is arranged downstream of the light detector (6).

11. Method according to claim 10, **characterised by** partially covering the measuring beam (3) or the reference beam (4) in the third position (P3, P3') of the cover device (5) or covering an additional light beam (L) emitted by the light source (2) as a reference beam (7), which light beam is intended to bypass the fluid (F), differently in the third position (P3") of the cover device (5) in comparison to the first position (P1) and to the second position (P2) of the cover device (5).

12. Method according to claim 11, **characterised by** covering the second reference beam (7) in the first position (P1) and in the second position (P2) of the cover device (5) and at least partially exposing the second reference beam (7) in the third position (P3") of the cover device (5).

13. Method according to either claim 10 or claim 12, **characterised by** comparing the output parameter of the light detector (6) in the third position (P3, P3', P3") of the cover device (5) with the output parameter of the light detector (6) in the first position (P1) or the second position (P2) of the cover device (5) in a processing unit (8) arranged downstream of the light detector (6) and outputting an indication of a blockage in the movement of the cover device (5) by means of the processing unit (8) if the difference between the compared output parameters falls below a predetermined threshold value.

14. Method according to claim 13, **characterised by** outputting the indication of a blockage in the movement of the cover device (5) only when the difference between the compared output parameters falls below the predetermined threshold value several times in succession.

## Revendications

1. Dispositif (1) pour la détection de propriétés d'un fluide (F) à examiner, avec une source de lumière (2) permettant d'émettre plusieurs rayons lumineux (L), un des rayons lumineux (L) étant un rayon de mesure (3) qui est conçu pour traverser le fluide (F) et un autre rayon lumineux (L) étant un rayon de référence (4), qui est conçu pour contourner le fluide (F), avec un dispositif de recouvrement mobile (5) branché en aval de la source de lumière (2), qui est conçu pour le recouvrement des rayons lumineux (L) et qui est disposé de façon à pouvoir être commuté entre une première position (P1) dégageant le rayon de mesure (3) et recouvrant le rayon de référence (4) et une deuxième position (P2) recouvrant le rayon de mesure (3) et dégageant le rayon de référence (4), et avec un photodétecteur (6) branché en aval du dispositif de recouvrement (5), dans lequel le dispositif de recouvrement (5) est disposé de façon à pouvoir être commuté dans une troisième position (P3, P3', P3"), dans laquelle les rayons lumineux (L) émis par la source de lumière (2) sont recouverts différemment par rapport à la première position (P1) et à la deuxième position (P2) du dispositif de recouvrement (5), ce qui fait qu'un paramètre de sortie du photodétecteur (6) influencé par les rayons lumineux (L) est différent, dans la troisième position (P3, P3', P3"), du paramètre de sortie du photodétecteur (6) dans la première position (P1) et dans la deuxième position (P2) du dispositif de recouvrement (5), **caractérisé en ce qu'**une unité de traitement (8), branchée en aval du photodétecteur (6), est prévue, qui est conçue pour la reconnaissance d'un blocage de mouvement du dispositif de recouvrement (5) grâce à une comparaison des paramètres de sortie du photodétecteur (6) dans la première position (P1), la deuxième position (P2) et la troisième position (P3, P3', P3") du dispositif de recouvrement (5).

2. Dispositif (1) selon la revendication 1, **caractérisé en ce que**, dans la troisième position (P3, P3') du dispositif de recouvrement (5), le rayon de mesure (3) ou le rayon de référence (4) est recouvert partiellement ou un rayon lumineux (L) supplémentaire émis par la source de lumière (2), en tant que deuxième rayon de référence (7), qui est conçu pour contourner le fluide (F), est recouvert différemment, dans la troisième position (P3") du dispositif de recouvrement (5), par rapport à la première position (P1) et à la deuxième position (P2) du dispositif de recouvrement (5).

3. Dispositif (1) selon la revendication 2, **caractérisé en ce que** le deuxième rayon de référence (7) est recouvert, dans la première position (P1) et dans la deuxième position (P2) du dispositif de recouvrement (5) et est au moins partiellement dégagé dans la troisième position (P3") du dispositif de recouvrement (5).

4. Dispositif (1) selon l'une des revendications 1 à 3, **caractérisé en ce que** le dispositif de recouvrement (5) est disposé de manière pivotante.

5. Dispositif (1) selon l'une des revendications 1 à 4, **caractérisé en ce que** le dispositif de recouvrement (5) est relié avec un moteur pas-à-pas (9).

6. Dispositif (1) selon l'une des revendications 1 à 5, **caractérisé en ce que** le dispositif de recouvrement (5) fait partie d'un sélecteur de rayon (10) disposé dans le trajet des rayons lumineux (L), qui comprend une plaque de recouvrement fixe (11) avec des ouvertures de passage (12) pour les rayons lumineux (L) et le dispositif de recouvrement (5) est disposé de manière mobile pour le recouvrement réglable des ouvertures de passage (12).

7. Dispositif (1) selon l'une des revendications 1 à 6, **caractérisé en ce que** le dispositif de recouvrement (5) est constitué d'un seul élément de recouvrement (5a).

8. Dispositif (1) selon la revendication 6 et 7, **caractérisé en ce que** l'élément de recouvrement (5a) est constitué d'une plaque mobile (5b) dans un plan parallèle au plan de la plaque de recouvrement fixe (11) du sélecteur de rayon (10), cette plaque mobile (5b) comprenant une ouverture de passage (13) pour un des rayons lumineux (L).

9. Dispositif (1) selon l'une des revendications 1 à 8, **caractérisé en ce que** l'unité de traitement (8) est conçue pour l'émission d'un message d'erreur.

10. Procédé de détection de propriétés d'un fluide (F) à examiner, grâce à l'émission de plusieurs rayons lumineux (L) par une source de lumière (2), un des rayons lumineux (L) étant un rayon de mesure (3) qui est conçu pour traverser le fluide (F) et un autre rayon lumineux (L) étant un rayon de référence (4), qui est conçu pour contourner le fluide (F), au contrôle d'un dispositif de recouvrement (5) branché en aval de la source de lumière (2), pour le déplacement vers une première position (P1) dégageant le rayon de mesure (3) et recouvrant le rayon de référence (4) et la détection des rayons lumineux (L) que le dispositif de recouvrement (5) a laissé passer avec un photodétecteur (6) branché en aval du dispositif de recouvrement (5) et contrôle du dispositif de recouvrement mobile (5) pour le déplacement vers une deuxième position (P2) recouvrant le rayon de mesure (3) et dégageant le rayon de référence (4) et la détection des rayons lumineux (L) que le dispositif de recouvrement (5) a laissé passer avec le photodétecteur (6), **caractérisé par** le contrôle du dispositif de recouvrement mobile (5) pour le déplacement vers une troisième position (P3, P3', P3") et la détection des rayons lumineux (L) que le dispositif de recouvrement (5) a laissé passer avec le photodétecteur (6), les rayons lumineux (L) émis par la source de lumière (2) étant recouverts dans cette troisième position (P3, P3', P3") différemment par rapport à la première position (P1) et à la deuxième position (P2) du dispositif de recouvrement (5), ce qui fait qu'un paramètre de sortie du photodétecteur (6) influencé par les rayons lumineux (L) est différent, dans la troisième position (P3, P3', P3"), du paramètre de sortie du photodétecteur (6) dans la première position (P1) et dans la deuxième position (P2) du dispositif de recouvrement (5), et une comparaison des paramètres de sortie du photodétecteur (6) dans la première position (P1), la deuxième position (P2) et la troisième position (P3, P3', P3") du dispositif de recouvrement (5), est effectuée avec une unité de traitement (8), branchée en aval du photodétecteur (6), pour la reconnaissance d'un blocage de mouvement du dispositif de recouvrement (5).

11. Procédé selon la revendication 10, **caractérisé par** le recouvrement partiel du rayon de mesure (3) ou du rayon de référence (4) dans la troisième position (P3, P3') du dispositif de recouvrement (5) ou recouvrement d'un rayon lumineux supplémentaire (L) émis par la source de lumière (2) en tant que deuxième rayon de référence (7), qui est conçu pour contourner le fluide (F), dans la troisième position (P3") du dispositif de recouvrement (5), différent par rapport à la première position (P1) et à la deuxième position (P2) du dispositif de recouvrement (5) .

12. Procédé selon la revendication 11, **caractérisé par** le recouvrement du deuxième rayon de référence (7) dans la première position (P1) et dans la deuxième position (P2) du dispositif de recouvrement (5) et dégagement au moins partiel du deuxième rayon de référence (7) dans la troisième position (P3") du dispositif de recouvrement (5).

13. Procédé selon l'une des revendications 10 à 12, **caractérisé par** la comparaison du paramètre de sortie du photodétecteur (6) dans la troisième position (P3, P3', P3") du dispositif de recouvrement (5) avec le paramètre de sortie du photodétecteur (6) dans la première position (P1) ou la deuxième position (P2) du dispositif de recouvrement (5) dans une unité de traitement (8) branchée en aval du photodétecteur (6) et au moyen de l'unité de traitement (8), émission d'une instruction concernant un blocage du mouvement du dispositif de recouvrement (5) lorsque la différence entre les paramètres de sortie comparés passe en dessous d'une valeur seuil prédéterminée.

14. Procédé selon la revendication 13, **caractérisé par** l'émission d'une instruction concernant un blocage du mouvement du dispositif de recouvrement (5) lorsque la différence entre les paramètres de sortie comparés passe en dessous de la valeur seuil prédéterminée plusieurs fois de suite.
